# EUROPEAN PATENT SPECIFICATION

(11) **EP 3 003 347 B1**
(45) Date of publication and mention of the grant of the patent: **24.06.2020**
(21) Application number: 14731762.2
(22) Date of filing: 27.05.2014
(51) Int. Cl.: A61K 38/16

(54) **NOVEL DIAGNOSIS AND THERAPY**
NEUARTIGE DIAGNOSE UND THERAPIE
NOUVEAUX DIAGNOSTIC ET TRAITEMENT

(30) Priority: 28.05.2013 GB 201309498
(43) Date of publication of application: 13.04.2016
(73) Proprietor: Swansea University, Swansea, South Wales SA2 8PP (GB)
(72) Inventor: DAVIES, Jeffrey Stephen, Swansea, South Wales SA2 8PP (GB)
(74) Representative: Myint, Julie Marie
(86) International application number: PCT/GB2014/051608
(87) International publication number: WO 2014/191727

(56) References cited:
- EP-A1- 2 444 411
- LI ENDAN ET AL: "Ghrelin directly stimulates adult hippocampal neurogenesis: implications for learning and memory.", ENDOCRINE JOURNAL, vol. 60, no. 6, 15 February 2013 (2013-02-15), pages 781-789, XP002728043, ISSN: 1348-4540
- MANUEL D GAHETE ET AL: "Role of ghrelin system in neuroprotection and cognitive functions: Implications in Alzheimer's disease", PEPTIDES, vol. 32, no. 11, 26 September 2011 (2011-09-26), pages 2225-2228, XP028114470, ELSEVIER, AMSTERDAM, NL ISSN: 0196-9781, DOI: 10.1016/J.PEPTIDES.2011.09.019 [retrieved on 2011-10-01]
- R. M KIEWIET ET AL: "Effects of acute administration of acylated and unacylated ghrelin on glucose and insulin concentrations in morbidly obese subjects without overt diabetes", EUROPEAN JOURNAL OF ENDOCRINOLOGY, vol. 161, no. 4, 1 October 2009 (2009-10-01), pages 567-573, XP055060822, ISSN: 0804-4643, DOI: 10.1530/EJE-09-0339
- CHEN YING ET AL: "Ghrelin modulates insulin sensitivity and tau phosphorylation in high glucose-induced hippocampal neurons.", BIOLOGICAL & PHARMACEUTICAL BULLETIN, vol. 33, no. 7, 2010, pages 1165-1169, XP002728044, ISSN: 1347-5215
- DELHANTY P J D ET AL: "Ghrelin and glucose homeostasis", PEPTIDES, vol. 32, no. 11, 1 March 2011 (2011-03-01) , pages 2309-2318, XP028114482, ELSEVIER, AMSTERDAM, NL ISSN: 0196-9781, DOI: 10.1016/J.PEPTIDES.2011.03.001 [retrieved on 2011-03-09]
- CONG WEI-NA ET AL: "Ghrelin receptor signaling: a promising therapeutic target for metabolic syndrome and cognitive dysfunction.", NIH Public Access Author Manuscript , 1 November 2010 (2010-11-01), XP002728045, Retrieved from the Internet: URL:http://www.ncbi.nlm.nih.gov/pmc/articl es/PMC2967656/pdf/nihms231921.pdf [retrieved on 2014-08-07]
- ZHAO W Q ET AL: "Insulin resistance and amyloidogenesis as common molecular foundation for type 2 diabetes and Alzheimer's disease", BIOCHIMICA ET BIOPHYSICA ACTA. MOLECULAR BASIS OF DISEASE, vol. 1792, no. 5, 1 May 2009 (2009-05-01), pages 482-496, XP026048586, AMSTERDAM, NL ISSN: 0925-4439, DOI: 10.1016/J.BBADIS.2008.10.014 [retrieved on 2008-11-05]
- M. SATOU ET AL: "Identification and Characterization of Acyl-Protein Thioesterase 1/Lysophospholipase I As a Ghrelin Deacylation/Lysophospholipid Hydrolyzing Enzyme in Fetal Bovine Serum and Conditioned Medium", ENDOCRINOLOGY, vol. 151, no. 10, 1 October 2010 (2010-10-01), pages 4765-4775, XP055031522, ISSN: 0013-7227, DOI: 10.1210/en.2010-0412
- Patric J. D. Delhanty ET AL: "Unacylated Ghrelin Rapidly Modulates Lipogenic and Insulin Signaling Pathway Gene Expression in Metabolically Active Tissues of GHSR Deleted Mice", PLoS ONE, vol. 5, no. 7, 26 July 2010 (2010-07-26), page e11749, XP055505741, DOI: 10.1371/journal.pone.0011749
- Z. B. Andrews ET AL: "Ghrelin Promotes and Protects Nigrostriatal Dopamine Function via a UCP2-Dependent Mitochondrial Mechanism", THE JOURNAL OF NEUROSCIENCE, vol. 29, no. 45, 11 November 2009 (2009-11-11), pages 14057-14065, XP055505746, US ISSN: 0270-6474, DOI: 10.1523/JNEUROSCI.3890-09.2009
- KUMAR R ET AL: "Proghrelin peptides: Desacyl ghrelin is a powerful inhibitor of acylated ghrelin, likely to impair physiological effects of acyl ghrelin but not of obestatin", REGULATORY PEPTIDES, ELSEVIER SCIENCE BV, NL, vol. 164, no. 2-3, 24 September 2010 (2010-09-24), pages 65-70, XP027229300, ISSN: 0167-0115 [retrieved on 2010-07-06]
- TOBIAS J. ZIMMERMANN ET AL: "Boron-Based Inhibitors of Acyl Protein Thioesterases 1 and 2", CHEMBIOCHEM - A EUROPEAN JOURNAL OF CHEMICAL BIOLOGY., vol. 14, no. 1, 2 January 2013 (2013-01-02), pages 115-122, XP055504724, DE ISSN: 1439-4227, DOI: 10.1002/cbic.201200571
- GABRIELE SIEGEL ET AL: "A functional screen implicates microRNA-138-dependent regulation of the depalmitoylation enzyme APT1 in dendritic spine morphogenesis", NATURE CELL BIOLOGY, vol. 11, no. 6, 24 May 2009 (2009-05-24), pages 705-716, XP055505631, GB ISSN: 1465-7392, DOI: 10.1038/ncb1876
- MARION RUSCH ET AL: "Identification of Acyl Protein Thioesterases?1 and 2 as the Cellular Targets of the Ras-Signaling Modulators Palmostatin?B and M", ANGEWANDTE CHEMIE INTERNATIONAL EDITION, vol. 50, no. 42, 10 October 2011 (2011-10-10), pages 9838-9842, XP055505636, ISSN: 1433-7851, DOI: 10.1002/anie.201102967
- MINHO MOON ET AL: "Ghrelin Regulates Hippocampal Neurogenesis in Adult Mice", ENDOCRINE JOURNAL, vol. 56, no. 3, 1 January 2009 (2009-01-01), pages 525-531, XP055505641, JP ISSN: 0918-8959, DOI: 10.1507/endocrj.K09E-089
- DELHANTY PATRIC J ET AL: "Des-acyl ghrelin: a metabolically active peptide", ENDOCRINE DEVELOPMENT, KARGER, CH, vol. 25, 1 January 2013 (2013-01-01), pages 112-121, XP009179461, ISSN: 1421-7082
- ZEENAT ATCHA ET AL: "Cognitive enhancing effects of ghrelin receptor agonists", PSYCHOPHARMACOLOGY, SPRINGER, BERLIN, DE, vol. 206, no. 3, 4 August 2009 (2009-08-04), pages 415-427, XP019736505, ISSN: 1432-2072, DOI: 10.1007/S00213-009-1620-6
- FUKATA YUKO ET AL: "Protein palmitoylation in neuronal development and synaptic plasticity", NATURE REVIEWS. NEUROSCIENCE, NATURE PUBLISHING GROUP, GB , vol. 11, no. 3 1 March 2010 (2010-03-01), pages 161-175, XP009507884, ISSN: 1471-003X, DOI: 10.1038/NRN2788 Retrieved from the Internet: URL:https://epo.summon.serialssolutions.co m/2.0.0/link/0/eLvHCXMwtV1Nb9QwELVokRAX1PL VpbTygWuEHHvj5FhV7MKhUKFFoqcojp1LYVntx6H_n je2403UIsGBS7QayfFq5mX8xh_PjL0zrtDd1LQZPmq XKY2C1WAYyUgHJS8LN60amoe8uVSLuf46p9sa-uOKe 9t_jTRsiDWdnP2HaKeXwoDfiDmeiDqefxX3axJe8IK pP37ia70Le91oWsNLVxL5tPuNQn7tYHO3bFYk3LoCl aZd1tvRWu9
- YOUNG FIONA B ET AL: "Putting proteins in their place: Palmitoylation in Huntington disease and other neuropsychiatric diseases", PROGRESS IN NEUROBIOLOGY, vol. 97, no. 2, 7 December 2011 (2011-12-07), pages 220-238, XP028917075, ISSN: 0301-0082, DOI: 10.1016/J.PNEUROBIO.2011.11.002

## Description

The present invention relates to the treatment of age-related cognitive decline and other CNS disorders and also to diagnostic biomarkers that are indicative of such diseases. Compounds for use in the treatment and diagnostic tests for the biomarkers form further aspects of the invention.

The gut hormone, Ghrelin, is known to be a 28 amino acid peptide with a unique octanoyl modification on Ser-3 (Kojima M et al., Nature 1999, 402: 656-660). It has been identified as an endogenous ligand for the growth hormone secretagogue receptor type 1a (GHS-R 1a), a G-protein coupled receptor.

It is released from the stomach during negative energy balance to re-establish neutral energy balance i.e by feeding, lipogenesis, inhibition of muscle atrophy.

The pro-ghrelin peptide undergoes post-translational acylation, which involves the addition of an octanoic-acid side chain onto the 3^{rd} residue (Serine). This acylation event is essential for binding and activation of the cognate ghrelin receptor, GHS-R. The enzyme responsible for this modification was identified as ghrelin-O-acyl transferase (GOAT)(Yang et al. Cell, 2008, 132:387-396)(Gutierrez et al. PNAS, 2008, 105:6320-6325). The mature acyl-ghrelin peptide (28 a.acids) is de-acylated (and therefore deactivated) by Acyl Protein Thioesterase 1 (APT1), also known as Lysophospholipase I (Shanando et al. 2004, Biochemical and Biophysical Research Communications, 325, 1487-1494) (Satou et al. Endocrinology, 2010, 151 (10) 4765-4775). It has also been reported that other enzymes such as butyrylcholinesterase and platelet-activating factor acetylhydrolase may cause de-acylation of ghrelin (May et al. 2009, GallusReactome REACT_19189.1).

Ghrelin has been reported as having potential clinical applications in the treatment of a wide range of diseases. These include metabolic disorders such as the regulation of energy balance and/or food intake, treatment of adipogenesis, adiposity and/or Obesity and Reduction of Body Weight, but other potential applications are in the treatment of tumors, inflammatory disease, cachexia, diabetes, the treatment of addiction processes, gastric postoperative ileus, and postoperative ileus.

Ghrelin mimetics and GHS-R agonists have been developed to treat conditions such as cachexia and endocrine disorders.

Recently, acyl-ghrelin, which is elevated during calorie restriction was shown to cross the blood-brain barrier, bind to ghrelin receptors (growth hormone secretagogue receptor, *Ghsr*) within the hippocampus, promote long-term potentiation and improve performance in spatial learning tasks (Carlini et al. Biochemical and Biophysical Research Communications, 2004, 313, 635-641; Diano et al. Nature Neuroscience, 2006, 9,3;381-388). Furthermore, ghrelin increases cell proliferation in the hippocampus (Moon et al. Endocrine Journal, 2009, 56,3, 525-531) and adult ghrelin deficient mice show reduced rates of new neuron differentiation that was restored to wild-type levels following ghrelin treatment (Li et al. Endocrine Journal, 2013, doi: 10.1507/endocrj. EJ13-0008).

The role of ghrelin in the CNS disorders such as Alzheimer's disease has also been reported. Gahete et al. Peptides (2011) 32 (11):2225-2228 reviews work in this area. This review paper reports that in relation to neuroprotection, ghrelin acted as a survival factor in-vitro for cortical neuronal cells by inhibiting apoptotic pathways regardless of its acylation. It is further noted that GOAT was expressed in the temporal lobe of the brain and that this was impaired in AD, suggesting that changes in locally-produced acylated/un-acylated ghrelin and In2-ghrelin variant may be of patho-physiological relevance.

The applicants have found that the peripheral administration of acyl-ghrelin has significant potential to treat cognitive decline/dementia and ameliorate neurodegeneration. Importantly, the applicants have also found that unacylated-ghrelin (UAG) may exacerbate cognitive impairment. Therefore, elevating the ratio of circulating acyl-ghrelin:UAG (AG:UAG), represents a therapeutic strategy to prevent or treat age-related cognitive decline and other CNS disorders.

This can be achieved by inhibiting enzymes that cause de-acylation of AG, including in particular Acyl Protein Thioesterase 1.

According to the present invention there is taught herein a ghrelin de-acylation inhibitor (GDAi) for use in the treatment or prophylaxis of physiological or pathophysiologicial conditions that are mediated by acyl-ghrelin (AG) and unacylated ghrelin (UAG) in mammals.

A particular GDAi for use herein is Acyl Protein Thioesterase 1 (APT1), but it is possible that inhibition of other enzymes that cause ghrelin de-acylation, such as APT2 and those described above may also be effective in this context. In particular, compounds which inhibit both APT1 and APT2 may be used.

Physiological or pathophysiological conditions that are mediated by acylated and unacylated ghrelin are those in which both acylated and unacylated ghrelin have an impact on the condition. In particular, the condition is one which is treated by the application of acylated gherlin, but exacerbated by the administration of unacylated ghrelin. Thus the condition is one which may be treated by enhancing the relative amount of acyl-ghrelin as compared to unacylated ghrelin. The applicants are the first to appreciate that the ratio of circulating AG:UAG may be a factor in disease mediation.

Such conditions may be identified using *in vivo* or *in vitro* tests, to show that AG and UAG produce opposing effects. For instance, the tests can be carried out *in vivo* using animal models or *in vitro.* In the former case, use may be made of transgenic animal models such as ghrelin-receptor null (ghsr^{-/-})mice. Separate administration of acylated ghrelin and unacylated ghrelin to such animals will allow a comparison of the relative effects on any organ or in any test to determine whether AG produces a beneficial effect and UAG produces a detrimental effect. An example of such a test is illustrated hereinafter.

Similar basic principles may be applied to *in-vitro* tests which may be carried out for instance using cell cultures as well as isolated reagents such as enzymes or receptors. Suitable tests will be available to the skilled person working in the particular field. Different and contrasting results following the administration of AG and UAG will be indicative that the condition is amenable to treatment in accordance with the invention. An example of such a test, showing opposing effects of AG and UAG on glucose output by primary hepatocytes has been reported previously (Gauna C et al. J.Clin.Endocrinol.Metab. 2005, 90(2):1055-1060).

In particular, such conditions may be mediated by growth hormone secretagogue (GHS) receptors. Thus conditions which are affected by agonists and/or antagonists of such receptors may be treated in accordance with the teachings herein. In particular, the GDAi used in the invention binds the APT-1 active site.

Use of ghrelin de-acylation inhibitors (GDAi), in particular those that block Lysophospholipase I / Acyl Protein Thioesterase 1 (APT1) activity in the circulation, enhance the relative level of acyl-ghrelin, thus improving its bioavailability and thus producing the required effect.

In a particular teaching, the mammals are humans but the inhibitors may also be employed in veterinary applications.

This therapeutic approach may be useful in the prophylaxis or therapy of the ghrelin-related clinical applications discussed above, such as Parkinson's disease, Alzheimer's disease, mild cognitive impairment (MCI), frontotemporal dementia, dementia, multiple sclerosis, motor neurone disease, Huntingdon's disease, epilepsy, anxiety disorders (including panic disorders and post-traumatic stress disorder (PTSD)), depression, alcohol disorder, drug abuse, growth retardation, cachexia, short-, medium-, and/or long term regulation of energy balance, short-, medium-, and/or long term regulation (stimulation and/or inhibition) of food intake, intake of rewarding food, adipogenesis, adiposity and/or obesity, body weight gain and/or reduction, diabetes, diabetes type I, diabetes type II, inflammatory effects, such as neuro-inflammation, gastric postoperative ileus, postoperative ileus and tumor cell proliferation in particular where the tumor is not one that includes an oncogenic mutation in a Ras gene, including the H-ras, N-ras, or K-ras genes that are frequently found in human tumors.

In a particular embodiment, the physiological or pathophysiological condition that is treated in accordance with the teachings herein are Central Nervous System (CNS) disorders, such as an age-related disorder. Particular examples include Parkinson's disease, Alzheimer's disease, frontotemporal dementia, dementia, multiple sclerosis, motor neurone disease, Huntingdon's disease, epilepsy, anxiety disorders (including panic disorders and post-traumatic stress disorder (PTSD)), depression, alcohol disorder, and drug abuse.

In particular, the inhibitor as taught herein is used in the treatment of Alzheimer's disease.

Suitable inhibitors are known in the art or can be prepared using known technology. APT1 inhibitors are known in the art for the treatment of Ras protein related diseases including cancer. They are described for example in Hedberg et al. Angew Chem Int Ed Engl (2012) 50 (42) 9832-7, Zimmermann et al. ChemBioChem 2013, 14, 115-122 and Dekker et al. Nature Chemical Biology, 2010, 6, 449-456.

Particular examples include small molecules including a range of lactone derivatives including Palmostatin B, Palmostatin M and tetrahydolipstatin as well as a range of boron-based compounds (i.e. organic compounds that contain at least one boron atom) such as boronic acids or borinic acids and derivatives thereof. Particular examples of boron-based compounds include phenylboronic acids where R is an optionally substituted phenyl group, as well as borinic acids or borinates of formula R¹R²B(OR³) where R¹ and R² are independently selected from organic groups such as optionally substituted phenyl and R³ is hydrogen or an organic group such as an optionally substituted phenyl groups. Derivatives may include bis-borinic acid derivatives and bis-borinate ester structures.

The structure of Palmostatin B is shown below as compound of formula (I) and Palmostatin M is of structure (II).

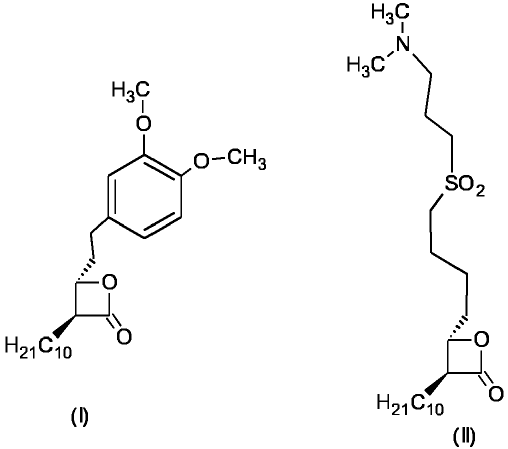

Suitable phenylboronic acids and derivatives which inhibit both APT1 and APT2 include in particular acids of formula (III)-(VII) below, but other suitable compounds are shown in Zimmerman et al. supra., the content of which is incorporated herein by reference.

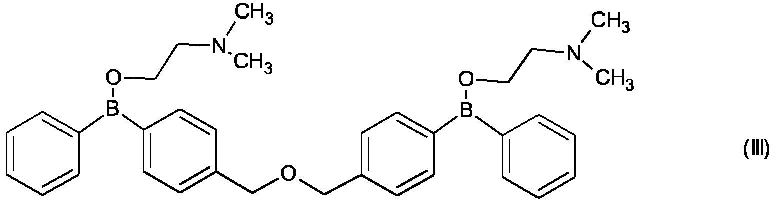

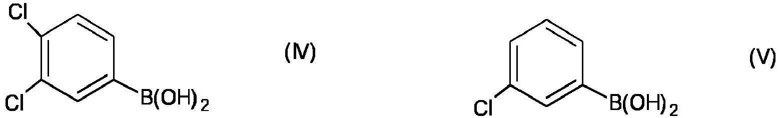

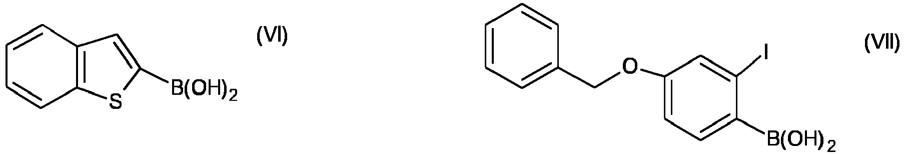

Alternatively, the inhibitor may be a specific binding partner for APT1. Particular specific binding partners are immunoglobulins, such as an antibody or a binding fragment thereof that bind an active site of APT1. Examples of suitable binding fragments of antibodies include Fab, Fab', F(ab)2, F(ab')2 and FV, VH and VK fragments. Antibodies may be polyclonal or monoclonal but in a particular embodiment, the antibodies are monoclonal antibodies.

Antibodies can be prepared using conventional methods involving inoculation of an animal such as mouse or guinea pig with APT1 in particular human APT1 or a fragment thereof which includes the active site. Thereafter, antibodies are harvested from the animal. Monoclonal antibodies can be obtained therefrom by fusing antibody producing cells such as spleen cells from the inoculated animal with a hybridoma cell, culturing this cell and harvesting monoclonal antibodies therefrom.

The ascribed sequence for APT1 is available as Uniprot number 075608 and characterised on protein database sequence (PDB) 1FJ2. The sequence is shown as SEQ ID NO 1.

The active site of APT1 has been identified as containing a catalytic triad of Ser-119, His-208 and Asp-174 in SEQ ID NO 1. Furthermore the crystal structure has been elucidated (Devedjiev et al. Structure, 8, 1137-1147 (2000). Thus fragments which contain the active site can be prepared.

As taught herein, the APT-1 inhibitor will generally be administered in the form of a pharmaceutical composition, in which the inhibitor is combined with a pharmaceutically acceptable carrier.

Suitable pharmaceutical compositions will be in either solid or liquid form. They may be adapted for administration by any convenient peripheral route, such as parenteral, oral, vaginal or topical administration or for administration by inhalation or insufflation. The pharmaceutical acceptable carrier may include diluents or excipients which are physiologically tolerable and compatible with the active ingredient. These include those described for example in Remington's Pharmaceutical Sciences, Mack Publishing Co. (A.R. Gennaro edit. 1985).

Parenteral compositions are prepared for injection, for example either subcutaneously, intramuscularly, intradermally, intravenously or via needle-free injection systems. They may be liquid solutions or suspensions, or they may be in the form of a solid that is suitable for solution in, or suspension in, liquid prior to injection. Suitable diluents and excipients are, for example, water, saline, dextrose, glycerol, or the like, and combinations thereof. In addition, if desired the compositions may contain minor amounts of auxiliary substances such as wetting or emulsifying agents, stabilizing or pH-buffering agents, and the like.

Oral formulations will be in the form of solids or liquids, and may be solutions, syrups, suspensions, tablets, pills, capsules, sustained-release formulations, or powders. Oral formulations include such normally employed excipients as, for example, pharmaceutical grades of mannitol, lactose, starch, magnesium stearate, sodium saccharin, cellulose, magnesium carbonate, and the like.

Topical formulations will generally take the form of suppositories, pessaries, intranasals sprays or aerosols, buccal or sublingual tablets or lozenges. For suppositories or pessaries, traditional binders and excipients may include, for example, polyalkylene glycols or triglycerides; such suppositories or pessaries may be formed from mixtures containing the active ingredient. Other topical formulations may take the form of a lotion, solution, cream, ointment or dusting powder, that may optionally be in the form of a skin patch.

Thus in a further aspect, there is taught herein a method for preventing or treating a physiological or pathophysiologicial conditions that are mediated by acyl-ghrelin and un-acylated ghrelin in mammals, which method comprises the peripheral administration to a patient in need thereof an effective amount of Ghrelin de-acylation inhibitor (GDAi) such as an inhibitor of APT1.

Suitable physiological or pathophysiological conditions are as described above. Similarly, the inhibitor is suitably administered in the form of a pharmaceutical composition as described above.

The amount of inhibitor administered will vary depending upon factors such as the specific nature of the inhibitor used, the size and health of the patient, the nature of the condition being treated etc. in accordance with normal clinical practice. Typically, a dosage in the range of from 0.01-1000 mg/Kg, for instance from 0.1-10mg/Kg, would produce a suitable therapeutic or protective effect.

Dosages may be given in single dose regimes, split dose regimes and/or in multiple dose regimes lasting over several days. Effective daily doses will, however vary depending upon the inherent activity of the active ingredient, such variations being within the skill and judgment of the physician.

The inhibitor as taught herein may be used in combination with one or more other active agents, such as one or more pharmaceutically active agents.

However, the applicants have also found that the ratio of circulating acylated ghrelin (AG) to un-acylated ghrelin (UAG) is an indicator or predictor of CNS disorder such as Parkinson's or Alzheimer's disease. This means that this ratio may be a useful early diagnostic biomarker for the diagnosis of such disease or a predisposition to such disease. This may be particularly useful since the early diagnosis of such disease is difficult before clinical signs, such as cognitive and motor dysfunction, become apparent. Of note is the fact that Parkinson's disease patients lose approximately 70% of their nigro-striatal dopaminergic neurons prior to the onset of motor symptoms. Thus a test that would allow early diagnosis would be extremely valuable and may allow for early prophylactic or therapeutic treatments to be administered.

Thus a further aspect of the invention provides an in vitro method for diagnosing or detecting a predisposition to a cognitive decline or dementia, which method comprises:
(i) measuring the amount of total ghrelin present in a biological sample;
(ii) measuring the amount of acylated ghrelin present in said sample;
(iii) calculating the ratio of acylated to un-acylated ghrelin present in the sample from the results of step i) and step ii); and
where the ratio of acylated to un-acylated ghrelin in the biological sample indicates the amount of acylated ghrelin is less than the amount of un-acylated ghrelin concluding said individual has or has a predisposition to developing cognitive impairment or dementia.

The method may be used as part of a method of staging a CNS disorder. In this embodiment, the results obtained are related to the stage of progression of a CNS disorder. Such staging may be advantageous for or used in the stratification of patients in clinical trials or directing the type of clinical treatment optimal for the stage of the CNS disorder.

In a particular embodiment, the method is used to measure the rate of progression of a CNS disorder, where the method is repeated periodically and the changing results are related to the rate of progression of a CNS disorder.

Biological samples used in the measurement will suitably comprise a circulatory sample such as a blood, plasma or serum sample, as well as other samples such as urine, cerebrospinal fluid or tissue samples.

Step (i) is suitably effected using a immunoassay method and kits for carrying out such a method are available commercially from Millipore (USA). Step (ii) may be effected by assaying specifically for acyl groups, and a kit for carrying out such a method based upon a double-antibody sandwich technique is available from Millipore (USA) and is described in Granata et al. 2007, Endocrinology, 148:2:512-519 and Falken et al. 2011 Journal of Clinical Endocrinology and Metabolism 96: 2227-35.

Care needs to be taken during this method to ensure that the integrity of the acyl group in AG is protected. This may be done for instance by careful handling of the sample, for instance by collection of sample into EDTA/Aprotinin tubes which are suitably stored at 4°C prior to analysis.

Typically the total amount of circulating ghrelin in a healthy human adult has been reported as being in the range of 250-800 pg/ml (Cummings et al.2001, Diabetes, 50, 8, 1714-1719). However, overnight fasting increases this level, with levels in Prader-Willi syndrome being measured at 1900 pg/ml (Cummings et al.2002, Nature Medicine, 8, 7, 643-644). Irrespective of the concentration however, the AG will generally represent at least 10% of the total ghrelin present and generally not more that 30%, for instance 25% of the total. Typically, the relative amounts of ratio of AG:UAG in a healthy human adult will be in the range of from 1 in 10 to 1 in 2 for example from 1 in 10 to 1 in 3. Any variation in this, in particular any significant decrease in the relative amount of AG present below 10% of the total may be indicative of the presence of CNS disease.

Alternatively, since the ratio of AG to UAG will be dependent upon de-acylation activity, the concentration or level of ghrelin de-acylating enzymes (GDA) such as APT1 or the de-acylation activity level of a sample as described above may provide a diagnostic biomarker for diagnosing such disease. The concentration or level of GDA enzymes may be detected by various techniques, including for instance quantitative ELISA techniques using antibodies which recognize the specific enzymes such as APT1 or quantitative polymerase chain reaction (qPCR), designed to measure the amount of mRNA encoding the GDA and in particular APT1 present in the sample.

De-acylation activity may be assayed by contacting the sample with a known concentration of AG and thereafter detecting the amount of residual AG. An example of such a method is described in Satou et al. supra.

Thus according to a further aspect, the invention provides a method for diagnosing or detecting a predisposition to a CNS disorder, which method comprises either measuring the amount of ghrelin de-acylation enzyme, in particular APT1 in a biological sample; or measuring the total ghrelin de-acylation activity of a biological sample; and relating the result to the presence or absence of CNS disorder or a predisposition thereto.

Measurement of the amount of GDA enzyme such as in particular APT1 may be a more convenient method for carrying out the diagnosis than measuring ratios of AG:UAG since the methodology doesn't require such careful sample preparation. In particular, where a AG:UAG ratio analysis is carried out, there is a need to ensure that the integrity of the acyl group is protected and this may require careful sample handling as discussed above. However, measurement of enzyme may be carried out using more robust methodology, and indeed, an ELISA test kit for the GDA, APT1 is available commercially from Mybiosource Inc. USA (cat no. MBS911730). However, since early diagnosis of diseases such as Alzheimer's and Parkinson's diseases is a significant requirement, such tests may be worthwhile.

The concentrations of GDA enzyme or the level of GDA activity that will be indicative of CNS disease will depend upon a variety of factors such as the nature of the patient. They may be determined using routine clinical practice, for instance by comparing relative levels with those found in healthy individuals.

Suitable biological samples used in the method will be similar to those described above, but in particular will be circulating samples such as blood, plasma or serum.

Such methods may be used in staging a CNS disease, or may be repeated periodically to monitor disease progression in a similar manner to the method described above.

In yet a further aspect, the invention provides the use of a ghrelin de-acylation enzyme and in particular APT1 as a diagnostic biomarker for CNS disease or a predisposition to CNS disease.

As a result, once CNS disease has been diagnosed using any of the methods described above, as taught herein a patient may be treated with a view to restoring the ratio of AG to UAG to within the healthy range as described above. For example, a GDAi may be administered to a patient diagnosed with CNS disease or with a predisposition to a CNS disorder in an amount which is effective to produce a level of circulating AG which is in the range of from 10-50%, for example from 10-30% by weight of the total circulating ghrelin. In particular, this will involve the administration of an ATP1 inhibitor, such as a Palmostatin, also as described above, to ensure that the conversion of AG to UAG is limited in the patient. Effective doses and dosage regimes will be determined according to clinical practice. Typically however, a dosage of ATP1 inhibitor as described above will be administered to a patient in need thereof, and then the level of circulating AG:UAG will continue to be monitored using the techniques described above, for example after from 7-28 days of the treatment, to ensure that the dosage is appropriate to achieve the desired effect. Where this is not the case, the dosage of the ATP1 inhibitor may be administered upwardly or downwardly as necessary.

As described in the examples hereinafter, the applicants found that elevating peripheral acyl-ghrelin levels increased the number of new adult born neurons in the dentate gyrus and improved pattern separation learning. Moreover, impairments observed in hippocampal cell proliferation and the number of immature neurons was observed following intra-venous infusion of UAG. Together, these findings demonstrate a previously unknown function for acyl-ghrelin in enhancing adult hippocampal neurogenesis and pattern separation, and notably, an opposing role for UAG in this paradigm.

In summary, the applicants' findings can be summarized as follows:
- Ghsr is expressed in the SGZ of the adult dentate gyrus
- Ghrelin enhances cell proliferation in a ligand induced Ghsr-dependent manner
- UAG attenuates hippocampal cell proliferation and immature neuron formation
- Ghrelin enhances adult hippocampal neurogenesis
- Ghrelin enhances pattern separation

The birth and maturation of new neurons in the adult mammalian dentate gyrus (DG), termed adult hippocampal neurogenesis (AHN), is essential for distinguishing similar but distinct contexts (Clelland et al. Science (2009) 325, 210-213; Creer et al.PNAS 107:5 2367-2372; Sahay et al. Nature.(2011) 472:466-70; Nakashiba et al. Cell, (2012) 149, 188-201). This form of cognition, termed pattern separation, is impaired in anxiety-disorders (Kheirbek et al. Nature Neuroscience 15,1613-1620 (2012)) and dementia (Grews et la. The Journal of Neuroscience (2010) 30:37, 12252-62).

This combined with the applicants findings demonstrate a previously unknown function for ghrelin in enhancing adult hippocampal neurogenesis and pattern separation learning. As a result, ghrelin can provide the basis for a useful treatment in particular for cognitive decline and anxiety related disorders as discussed above.

The invention will now be particularly described by way of example with reference to the accompanying diagrammatic drawings in which:

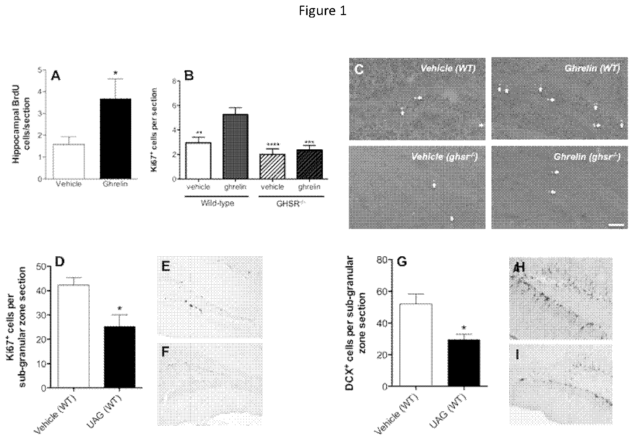

**Figure 1** shows the results of an experiment to show that Ghrelin enhances cell proliferation in the adult hippocampus. A) is a graph showing how a 7-day i.v. ghrelin infusion (80µg/day) increased the number of BrdU⁺ cells in adult rat hippocampus (BrdU (50mg/kg i.p) given 30 minutes prior to brain collection.*P<0.05 vs vehicle. B) is a graph showing the results of a 7-day treatment (48µg/day) increased Ki67⁺ cells in the DG of adult WT mice (**,P<0.01 vs ghrelin, 1 way-ANOVA followed by Bonferroni's post-hoc test). However, no corresponding increase was observed in the *ghsr*+ littermates, suggesting that the ghrelin-mediated increase in cell proliferation in WT mice was mediated by GHS-R. C) Photomicrographs of Ki67⁺ cells (indicated by white arrows) localized to the SGZ of the DG of *ghsr*⁺ and WT mice following treatment. Scale bar = 100µm. D) is a graph showing that a 7-day infusion of UAG (48µg/day) reduced the number of dividing cells (Ki67⁺). F) and G) immature neurons (DCX⁺; I) in the DG of adult wild-type mice compared to vehicle-treated controls (E,H) (*, P<0.05 vs veh). All data shown are mean + SEM. Statistical comparison by unpaired Student's t-test unless stated.

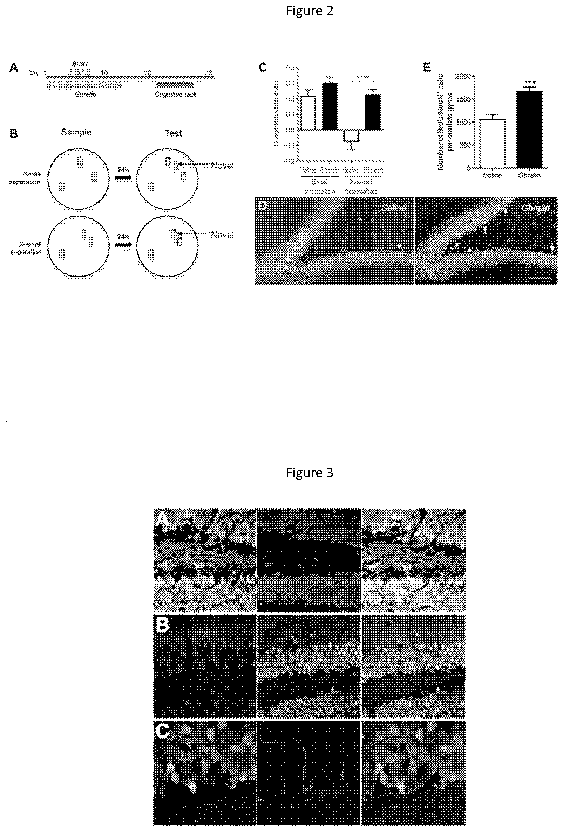

**Figure 2** shows the results of an experiment to demonstrate that ghrelin enhances spatial pattern separation in adult rats. A) Experimental paradigm B) Schematic of Spontaneous location recognition (SLR) task. During a sample phase animals were exposed to 3 identical objections (two were located close together (small separation) or very close together (X-small separation) and the 3^{rd} was further away) for 10 minutes. 24 hours later the rats were exposed to 2 of the objects during the test phase and the time spent exploring each object was measured. Dotted objects indicate the original location during the sample phase. C) Discrimination ratios during the test phase for 'novel' conditions in the SLR task. Both treatment groups showed enhanced exploratory behaviour for the 'novel' location in the small separation condition. However, only ghrelin treated rats showed enhanced exploratory behaviour for the 'novel' location in the X-small separation condition. ***p<0.0001, one-way repeated measures ANOVA followed by Bonferroni post-hoc comparisons, n=12 per group. D) Representative images of the new adult-born DG neurons (white arrows) co-expressing NeuN⁺ and BrdU⁺. E) Ghrelin treatment significantly increased the number of new adult-born neurons. ***p<0.001, unpaired t-test. Scale bar =100µm.
**Figure 3** shows how GHSR is expressed on granule cell neurons in the hippocampal dentate gyrus. A) GHSR is expressed on granule cell neurons of the adult DG. B) GHSR is co-expressed with the pro-neurogenic transcription factor Pax-6, but not with the neural stem cell marker, nestin (C).

### Example 1

### Examination of the role of Ghrelin in hippocampal cell proliferation

To further examine the role of ghrelin in enhancing cell proliferation in the SGZ, the effect of peripherally administered ghrelin in both wild-type and ghrelin-receptor null (ghsr^{-/-}) mice (Zigman et al. J. Clin. Invest (2005)115 (12:3564-72)) was analysed.

Adult growth hormone secretagogue receptor (GHSR)-null mice (8-10 weeks-old) and their wild-type littermates were used. These were derived from crosses between animals that were heterozygous for the GHSR-null allele and that had been backcrossed >10 generations onto a C57BL6/J genetic background. Metabolic aspects of these mice have been published (Davies et al. 2009 Molecular Endocrinology, 23(6), 914-24.).

Ghrelin was administered peripherally via an intravenous osmotic mini-pump at a rate of 1 µl/hour with a total of 48µg/day to groups of 6 mice. Control groups were given peripheral saline.

After 7 days, mice were killed by cervical dislocation whole brain was removed and immediately snap frozen on dry ice.

Cell proliferation in the hippocampal dentate gyrus was subsequently analysed using a one-in-fifteen series of 10µm sections (150µm apart) from each animal. These were stained for Ki67 immunofluorescence and a primary antibody rabbit anti-Ki67 (1:500 Abcam) applied. Immunolabelled cells were counted throughout the rostro-caudal extent of the granule cell layer and quantified.

Immunolabelled cells were counted bilaterally using a x40 objective (Zeiss Axioscope) throughout the rostro-caudal extent of the granule cell layer (GCL). Resulting numbers were divided by the number of coronal sections analyzed and multiplied by the distance between each section to obtain an estimate of the number of cells per hippocampus (and divided by 2 to obtain the total per dentate gyrus).

All experiments and analyses were performed blind to genotype and treatment.

Statistical analyses were carried out using GraphPad Prizm 6.0 for Mac. Statistical significance was assessed by unpaired two-tailed Student's t-test or one-way ANOVA with Bonferroni's post-hoc test unless described otherwise: *, *P*<0.05; **, *P*<0.01; ***, *P*<0.001.
The results are shown in Figure 1.

Ghrelin treated wild-type mice showed a significant increase in cell proliferation in the sub-granular zone of the dentate gyrus compared to saline treated mice (P<0.01, Fig 1B). In contrast, genetic blockade of ghrelin signaling in Ghsr^{-/-} mice inhibited the proliferative effect of ghrelin.

Of note, Ghsr-dependent cell proliferation may be further complicated by both ligand-dependent and ligand-independent signaling. However, basal hippocampal cell proliferation was not reduced in saline treated Ghsr^{-/-} mice compared to saline treated wild-type mice suggesting that constitutive Ghsr signaling may not be a primary regulator of SGZ cell proliferation.

### Example 2

### Effects of unacylated-ghrelin (UAG)

To determine whether unacylated-ghrelin (UAG) regulates NSPC plasticity, cell proliferation and immature neurons were quantified in wild-type mice following either peripheral administration of saline or UAG (i.v, 48ug/day, n=6/group) for 7-days. The method was similar to that described in Example 1.

UAG, which does not bind or activate Ghsr and blocks the orexigenic and GH-releasing action of acyl-ghrelin, attenuated hippocampal cell proliferation in wild-type mice (P<0.05, Fig ID). This was accompanied by a significant reduction in the number of immature DCX⁺ neurons within the SGZ following UAG treatment (P<0.05, Fig 1G).

This significant finding suggests that the circulating acyl-ghrelin:UAG ratio is an important modulator of adult neurogenesis and that it may be a novel biomarker of cognitive dysfunction.

### Example 3

### Effects of the ghrelin-mediated increase in hippocampal cell proliferation on numbers of mature adult born DG neurons and pattern separation learning

A study was carried out to see whether the ghrelin-mediated increase in dividing progenitor SGZ cells resulted in more mature adult born DG neurons and enhanced pattern separation learning.

All behavioural testing was carried out in accordance with the United Kingdom Animals (Scientific Procedures) Act 1986, under appropriate Home Office personal and project licenses in the University of Cambridge, Experimental Psychology.

Adult male Lister-hooded rats (n=12/group) were given daily injections of either saline or ghrelin (10µg/kg i.p) (days 1-14) and BrdU (50mg/kg i.p) on days 5-8 prior to analysis of spatial pattern separation using a spontaneous location recognition task (SLR) on days 22-26.

This dose of ghrelin was chosen as it has previously been shown to increase food intake and elevate plasma ghrelin concentrations to the same degree as a 24h fast in rats (Wren et al. Diabetes (2001)50(11) 2540-7). To reduce/avoid the acute LTP-mediated effects on pattern separation the final injection of ghrelin was given 7 days before the start of cognitive testing.

As expected, both saline and ghrelin treated groups were able to distinguish similar but distinct spatial locations, however, only ghrelin treated rats were able to distinguish between very similar spatial locations - consistent with enhanced pattern separation.

Animals were anesthetized with sodium pentobarbital and perfused transcardially with 0.9% NaCl solution, followed by 4% paraformaldehyde (PFA) in 0.1M phosphate buffer, pH 7.4. Brains were post-fixed in 4% PFA for 24h, and cryoprotected in 30% sucrose for 24h. The brains were then sectioned on a freezing-stage microtome into a 30µm thick coronal section series. Immunohistochemical staining was performed on free-floating sections.

For detection of BrdU, sections were pre-treated with 2N HCl for 30 mins at 37oC and washed in 0.1 M borate buffer, pH8.5, for 10 min. Immunofluorescent double-labeling was performed. Primary antibodies used were: mouse anti-BrdU (1:50 AbD Serotec, UK), goat anti-doublecortin (Santa Cruz Biotechnology, USA), mouse anti-NeuN (Chemicon, Temecula, CA, USA). Sections were also stained with Hoechst (Invitrogen) and mounted with prolong-gold anti-fade reagent (Invitrogen).

A one-in-twelve series of 30µm sections (360µm apart) from each animal was immunohistologically stained (as described in Example 1 above) and analysed by fluorescent and/or confocal microscopy. Immunolabelled cells were counted also as described in Example 1. Only cells with complete/strong BrdU immunoreactivity, oval nuclei, were counted. BrdU i.r cells that showed partial/weak i.r were omitted from the analysis as described by Eriksson et al.Nature Medicine 4, 1313-1317 (1998).

This analysis showed that ghrelin-treatment significantly increased the total number of new adult-born neurones (BrdU⁺/NeuN⁺) in the DG (P<0.001; Fig 2E). DG neuroblast number (DCX⁺) remained significantly higher in the ghrelin-treated group 14 days after the final injection of AG (P<0.01, data not shown).

This is the first demonstration that ghrelin enhances pattern-separation learning via a mechanism consistent with elevated AHN. The result is in keeping with the finding that elevating AHN is sufficient to increase pattern-separation (Sahay et al. Nature (2011) 472 (7344) 466-70).

### Example 4

### Effect of circulating ghrelin on NSPC plasticity

To determine whether circulating ghrelin may directly regulate NSPC plasticity the expression of Ghsr on cells of the sub-granular zone (SGZ) of the dentate gyrus was analysed. Adult GHSR-GFP transgenic mice (Andrews et al.2012 153(11), 5452-5466) were perfused and brains removed for immunohistochemical analysis (as above - Example 3) of neuronal, neural stem cell and Ghsr co-localisation. Tissues were subsequently analysed by confocal laser scanning microscopy in z-stack mode to prevent false-positive results.

The results, given in Figure 3, show that Ghsr is expressed throughout the DG, including the SGZ, and that Ghsr is expressed on DG neurons (GFP⁺/NeuN⁺, Figure 3A). These neurons were not co-expressed with type I and type II NSPCs (Sox-2⁺, nestin⁺ with radial processes; Figure 3B). However, a high proportion of GFP⁺ neurons were seemingly in contact with NSPC's. Notably, it appears that the transcription factor, paired box 6 (Pax-6), is co-expressed with Ghsr in the dentate gyrus of adult mice. Pax-6 has previously been shown to regulate proliferation and differentiation in these cells (Maekawa et al. Genes Cells (2005)10(10)1001-14) and promote early maturation of neuronal precursors and the frequency of neuronal phenotypes (Klempin et al. Stem Cells (2012) 30, 3, 500-509). This is consistent with the present findings that only 3-weeks after the first ghrelin injection rats showed enhanced ability in a pattern separation-dependent cognitive task and increased number of mature adult-born neurons (Example 3).

## Claims

1. An in vitro method for diagnosing or detecting a predisposition to a cognitive decline or dementia, which method comprises:
(i) measuring the amount of total ghrelin present in a biological sample;
(ii) measuring the amount of acylated ghrelin present in said sample;
(iii) calculating the ratio of acylated to un-acylated ghrelin present in the sample from the results of step i) and step ii); and
where the ratio of acylated to un-acylated ghrelin in the biological sample indicates the amount of acylated ghrelin is less than the amount of un-acylated ghrelin concluding said individual has or has a predisposition to developing cognitive impairment or dementia.

2. The method according to claim 1 wherein said method is for staging cognitive decline or dementia, which method comprises measuring the ratio of acylated to un-acylated ghrelin in a biological sample, and relating the result to the stage of progression of the cognitive decline or dementia.

3. The method according to claim 1 for measuring the rate of progression of cognitive decline or dementia, which method comprises measuring the ratio of acylated to un-acylated ghrelin in a biological sample periodically, and relating the changing result to the rate of progression of the cognitive impairment or dementia.

4. The method according to any one of claims 1 to 3 wherein the biological sample is a blood, plasma, serum, cerebrospinal fluid or urine sample.

5. The method for diagnosing or detecting a predisposition to cognitive decline or dementia according to any one of claims 1 to 4, which method comprises either measuring the amount of ghrelin de-acylation enzyme; or measuring the total ghrelin de-acylation activity of a biological sample.

6. The method according to claim 5 wherein the amount of ghrelin de-acylation enzyme is measured.

7. The method according to claim 6 wherein the ghrelin de-acylation enzyme is APT1.

## Patentansprüche

1. In-vitro-Verfahren zur Diagnose oder zum Detektieren einer Veranlagung zum kognitiven Abbau oder zu Demenz, wobei das Verfahren Folgendes umfasst:
(i) Messen der Menge des gesamten Ghrelins, das in einer biologischen Probe vorliegt;
(ii) Messen der Menge des acylierten Ghrelins, das in der Probe vorliegt;
(iii) Berechnen des Verhältnisses von acyliertem zu nicht acyliertem Ghrelin, das in der Probe vorliegt, aus den Ergebnissen von Schritt i) und Schritt ii); und
wobei das Verhältnis von acyliertem zu nicht acyliertem Ghrelin in der biologischen Probe anzeigt, dass die Menge von acyliertem Ghrelin kleiner ist als die Menge von nicht acyliertem Ghrelin, woraus geschlussfolgert werden kann, dass das Individuum eine kognitive Beeinträchtigung oder Demenz aufweist oder eine Veranlagung zu deren Entwicklung aufweist.

2. Verfahren nach Anspruch 1, wobei das Verfahren zur Einstufung eines kognitiven Abbaus oder einer Demenz gedacht ist, wobei das Verfahren das Messen des Verhältnisses von acyliertem zu nicht acyliertem Ghrelin in einer biologischen Probe und das Verknüpfen des Ergebnisses mit der Stufe des Fortschreitens des kognitiven Abbaus oder der Demenz umfasst.

3. Verfahren nach Anspruch 1 zum Messen des Fortschreitens eines kognitiven Abbaus oder einer Demenz, wobei das Verfahren das periodische Messen des Verhältnisses von acyliertem zu nicht acyliertem Ghrelin in einer biologischen Probe und das Verknüpfen des sich verändernden Ergebnisses mit dem Fortschreiten der kognitiven Beeinträchtigung oder der Demenz umfasst.

4. Verfahren nach einem der Ansprüche 1 bis 3, wobei die biologische Probe einer Blut-, Plasma-, Serum-, Rückenmarksflüssigkeits- oder Urinprobe entspricht.

5. Verfahren zur Diagnose oder zum Detektieren einer Veranlagung zum kognitiven Abbau oder zu Demenz nach einem der Ansprüche 1 bis 4, wobei das Verfahren entweder das Messen der Menge des Ghrelin-Deacylierungsenzyms; oder das Messen der gesamten Ghrelin-Deacylierungswirkung einer biologischen Probe umfasst.

6. Verfahren nach Anspruch 5, wobei die Menge des Ghrelin-Deacylierungsenzyms gemessen wird.

7. Verfahren nach Anspruch 6, wobei das Ghrelin-Deacylierungsenzym APT1 ist.

## Revendications

1. Méthode in vitro permettant de diagnostiquer ou de détecter une prédisposition à un déclin cognitif ou à la démence, ladite méthode comprenant :
(i) la mesure de la quantité de ghréline totale présente dans un échantillon biologique ;
(ii) la mesure de la quantité de ghréline acylée présente dans ledit échantillon ;
(iii) le calcul du rapport de ghréline acylée à ghréline non acylée présentes dans l'échantillon à partir des résultats de l'étape i) et de l'étape ii) ; et
dans laquelle le rapport de ghréline acylée à ghréline non acylée dans l'échantillon biologique indique que la quantité de ghréline acylée est inférieure à la quantité de ghréline non acylée, concluant ainsi que ledit individu est atteint ou a une prédisposition à développer une déficience cognitive ou la démence.

2. Méthode selon la revendication 1, dans laquelle ladite méthode est destinée à définir un stade de déclin cognitif ou de démence, ladite méthode comprenant la mesure du rapport de ghréline acylée à ghréline non acylée dans un échantillon biologique, et la mise en correspondance du résultat au stade d'évolution du déclin cognitif ou de la démence.

3. Méthode selon la revendication 1 destinée à mesurer la vitesse d'évolution du déclin cognitif ou de la démence, ladite méthode comprenant la mesure du rapport de ghréline acylée à ghréline non acylée dans un échantillon biologique de manière périodique, et la mise en correspondance du résultat de variation à la vitesse d'évolution de la déficience cognitive ou de la démence.

4. Méthode selon l'une quelconque des revendications 1 à 3 dans laquelle l'échantillon biologique est un échantillon de sang, de plasma, de sérum, de liquide céphalorachidien ou d'urine.

5. Méthode permettant de diagnostiquer ou de détecter une prédisposition à un déclin cognitif ou à la démence selon l'une quelconque des revendications 1 à 4, ladite méthode comprenant soit la mesure de la quantité d'enzyme de désacylation de la ghréline ; soit la mesure de l'activité de désacylation de la ghréline totale d'un échantillon biologique.

6. Méthode selon la revendication 5 dans laquelle la quantité d'enzyme de désacylation de la ghréline est mesurée.

7. Méthode selon la revendication 6 dans laquelle l'enzyme de désacylation de la ghréline est l'APT1.


### SEQUENCE LISTING

<110> Swansea University
<120> Novel Diagnosis and Therapy
<130> P3011/WO
<150> GB1309498.2
   <151> 2013-05-28
<160> 1
<170> BiSSAP 1.2
<210> 1
   <211> 230
   <212> PRT
   <213> Homo sapiens
<220>
   <223> Enzyme
<400> 1
   Met Cys Gly Asn Asn Met Ser Thr Pro Leu Pro Ala Ile Val Pro Ala
   1               5                   10                  15
   Ala Arg Lys Ala Thr Ala Ala Val Ile Phe Leu His Gly Leu Gly Asp
               20                  25                  30
   Thr Gly His Gly Trp Ala Glu Ala Phe Ala Gly Ile Arg Ser Ser His
           35                  40                  45
   Ile Lys Tyr Ile Cys Pro His Ala Pro Val Arg Pro Val Thr Leu Asn
       50                  55                  60
   Met Asn Val Ala Met Pro Ser Trp Phe Asp Ile Ile Gly Leu Ser Pro
   65                  70                  75                  80
   Asp Ser Gln Glu Asp Glu Ser Gly Ile Lys Gln Ala Ala Glu Asn Ile
                   85                  90                  95
   Lys Ala Leu Ile Asp Gln Glu Val Lys Asn Gly Ile Pro Ser Asn Arg
               100                 105                 110
   Ile Ile Leu Gly Gly Phe Ser Gln Gly Gly Ala Leu Ser Leu Tyr Thr
           115                 120                 125
   Ala Leu Thr Thr Gln Gln Lys Leu Ala Gly Val Thr Ala Leu Ser Cys
       130                 135                 140
   Trp Leu Pro Leu Arg Ala Ser Phe Pro Gln Gly Pro Ile Gly Gly Ala
   145                 150                 155                 160
   Asn Arg Asp Ile Ser Ile Leu Gln Cys His Gly Asp Cys Asp Pro Leu
                   165                 170                 175
   Val Pro Leu Met Phe Gly Ser Leu Thr Val Glu Lys Leu Lys Thr Leu
               180                 185                 190
   Val Asn Pro Ala Asn Val Thr Phe Lys Thr Tyr Glu Gly Met Met His
           195                 200                 205
   Ser Ser Cys Gln Gln Glu Met Met Asp Val Lys Gln Phe Ile Asp Lys
       210                 215                 220
   Leu Leu Pro Pro Ile Asp
   225                 230